(19) Europäisches Patentamt
European Patent Office
Office européen des brevets

(11) EP 3 070 117 B1

(12) **EUROPEAN PATENT SPECIFICATION**

(45) Date of publication and mention of the grant of the patent:
**08.08.2018 Bulletin 2018/32**

(21) Application number: **14868847.6**

(22) Date of filing: **12.11.2014**

(51) Int Cl.:
***C08J 7/18*** (2006.01) ***A61L 29/00*** (2006.01)
***C08F 2/00*** (2006.01) ***C08F 2/50*** (2006.01)
***C08F 279/02*** (2006.01)

(86) International application number:
**PCT/JP2014/079947**

(87) International publication number:
**WO 2015/087656 (18.06.2015 Gazette 2015/24)**

(54) **SURFACE MODIFICATION METHOD AND SURFACE-MODIFIED ELASTIC OBJECT**

OBERFLÄCHENMODIFIZIERUNGSVERFAHREN UND OBERFLÄCHENMODIFIZIERTER ELASTISCHER KÖRPER

PROCÉDÉ DE MODIFICATION DE SURFACE ET OBJET ÉLASTIQUE MODIFIÉ EN SURFACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2013 JP 2013255151**

(43) Date of publication of application:
**21.09.2016 Bulletin 2016/38**

(73) Proprietor: **Sumitomo Rubber Industries, Ltd.**
**Kobe-shi, Hyogo 651-0072 (JP)**

(72) Inventor: **MINAGAWA Yasuhisa**
**Kobe-shi**
**Hyogo 651-0072 (JP)**

(74) Representative: **Manitz Finsterwald Patentanwälte PartmbB**
**Martin-Greif-Strasse 1**
**80336 München (DE)**

(56) References cited:
**WO-A1-2012/165525** **WO-A1-2012/165525**
**CN-A- 101 565 489** **JP-A- H1 090 500**
**JP-A- H10 298 320** **JP-A- 2001 031 871**
**JP-A- 2001 046 956** **JP-A- 2001 046 956**
**JP-A- 2005 253 538** **JP-A- 2005 253 538**
**JP-A- 2009 226 718** **JP-A- 2009 226 718**
**JP-A- 2013 159 629** **JP-A- 2013 208 777**
**JP-A- 2013 237 802** **JP-A- 2013 237 802**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

TECHNICAL FIELD

The present invention relates to a surface modification method and surface-modified elastic bodies, including medical devices, e.g. a gasket for syringes, a catheter, a blood or body fluid analyzer, etc., at least part of whose surface is modified by the surface modification method.

BACKGROUND ART

In view of the importance of sealing properties, elastic bodies such as rubber are used in parts which slide while maintaining their sealing performance, for example a gasket which is integrated with a syringe plunger and forms a seal between the plunger and barrel. Unfortunately, such elastic bodies have a slight problem with sliding properties (see Patent Literature 1). To solve this problem, a sliding property improving agent, for example silicone oil, is applied to the sliding surface. However, a concern has been raised about the potential of silicone oil to accelerate adsorption or aggregation of proteins in recently marketed bio-preparations. On the other hand, gaskets not coated with a sliding property improving agent have poor sliding properties and thus do not allow the plungers to be smoothly pushed but cause them to pulsate during administration, leading to problems such as an inaccurate injection amount and infliction of pain on patients.

To satisfy the conflicting requirements, sealing properties and sliding properties, a coating technique using a self-lubricating PTFE film has been proposed (see Patent Literature 2). Unfortunately, such PTFE films are generally expensive and increase the production cost of processed products. Thus, the range of applications of these films is limited. Also, products coated with PTFE films might not be reliable when they are used in applications where durability is required as sliding or the like is repeated. Furthermore, since PTFE is vulnerable to electron beams and radioactive rays, the PTFE-coated products unfortunately cannot be sterilized by radiation.

Consideration may also be given to the use in other applications where sliding properties are required in the presence of water. Specifically, water can be delivered without a loss by reducing the fluid resistance of the inner surface of a pre-filled syringe or of the inner surface of a pipe or tube for delivering aqueous solutions, blood, or body fluid (including dilutions thereof), or by increasing or markedly reducing the contact angle with water. Moreover, catheters to be inserted into blood vessels or urethra need to have high sliding properties so as not to damage blood vessels or urethra or not to inflict pain on humans. Drainage of water on wet roads and of snow on snowy roads can be improved by reducing the fluid resistance of the groove surfaces of tires, or by increasing or markedly reducing the contact angle with water. This results in improved hydroplaning performance and improved grip performance and therefore better safety. Furthermore, less adhesion of dirt and dusts can be expected when the sliding resistance of the sidewall surfaces of tires or the walls of buildings is reduced, or when their contact angle with water is increased.

Further advantageous effects can be expected, including, for example: less pressure loss upon delivering fluids such as water or aqueous solutions through diaphragms such as diaphragm pumps or valves; easy sliding of skis and snowboards achieved by enhancing the sliding properties of the sliding surfaces thereof; better noticeability of road signs and signboards achieved by enhancing the sliding properties thereof to allow snow to readily slide on the surface; reduction in water resistance or drag and less adsorption of proteins and, therefore, less adhesion of bacteria on the outer peripheries of ships, achieved by reducing the sliding resistance of the outer peripheries or by increasing the contact angle with water; and reduction in water resistance or drag of swimsuits achieved by improving the sliding properties of the thread surfaces thereof.

WO 2012/165525 A1 and CN 101 565 489 A disclose a method for surface-modifying an object made of a rubber vulcanizate or a thermoplastic elastomer, the method comprising: step 1 of forming polymerization initiation points on a surface of the object and step 2 of radically polymerizing a monomer starting from the polymerization initiation points to grow polymer chains on the surface of the object.

JP 2001-046956 A discloses an active energy ray-curable coating composition for protecting the surface of a substrate such as paper, plastic, metal, glass, and ceramic-based inorganic materials.

CITATION LIST

PATENT LITERATURE

Patent Literature 1: JP 2004-298220 A
Patent Literature 2: JP 2010-142573 A

## SUMMARY OF INVENTION

### TECHNICAL PROBLEM

The present invention aims to solve the above problems and provide a method for surface-modifying a rubber vulcanizate or a thermoplastic elastomer. The method makes it possible to impart excellent sliding properties, excellent durability after repeated sliding, and excellent properties to prevent adsorption or aggregation of proteins, and further maintain good sealing properties for a long time, without using expensive self-lubricating resins. The present invention also aims to provide surface-modified elastic bodies, including medical devices, e.g. a gasket for syringes, a catheter, a blood or body fluid analyzer, etc., at least part of whose surface is modified by the surface modification method.

### SOLUTION TO PROBLEM

The present invention relates to a method for surface-modifying an object made of a rubber vulcanizate or a thermoplastic elastomer, the method including: step 1 of forming polymerization initiation points on a surface of the object; step 2 of radically polymerizing a monomer starting from the polymerization initiation points to grow polymer chains on the surface of the object; and step 3 of irradiating the grown polymer chains with an electron beam.

The rubber vulcanizate or thermoplastic elastomer preferably contains an allylic carbon atom which is adjacent to a double bond.

The polymerization initiation points are preferably formed by adsorbing a polymerization initiator onto the surface of the object.

The polymerization initiator is preferably at least one of a benzophenone compound or a thioxanthone compound.

Preferably, the adsorbed polymerization initiator is further chemically bonded to the surface of the object by irradiation with light.

The radical polymerization is preferably photoradical polymerization.

The photoradical polymerization preferably involves irradiation with light having a wavelength of 330 to 400 nm.

The radical polymerization preferably includes inserting an inert gas into a reaction vessel and a reaction solution to replace an atmosphere therein with the inert gas before or during the light irradiation, and then polymerizing the monomer.

The radical polymerization preferably includes evacuating a reaction vessel to remove oxygen before or during the light irradiation, and then polymerizing the monomer.

The electron beam is preferably at a dose of 1 to 500 kGy.

The monomer is preferably at least one selected from the group consisting of acrylic acid, acrylic acid esters, acrylamide, alkali metal salts of acrylic acid, amine salts of acrylic acid, methacrylic acid, methacrylic acid esters, methacrylamide, alkali metal salts of methacrylic acid, amine salts of methacrylic acid, dimethylacrylamide, diethylacrylamide, isopropylacrylamide, hydroxyethylacrylamide, acryloylmorpholine, dimethylmethacrylamide, diethylmethacrylamide, isopropylmethacrylamide, hydroxyethylmethacrylamide, methacryloylmorpholine, and acrylonitrile.

Preferably, the monomer or a solution thereof contains a polymerization inhibitor, and is polymerized in the presence of the polymerization inhibitor.

The polymerization inhibitor is preferably 4-methylphenol.

Preferably, the polymer chains each have a length of 10 to 50,000 nm.

The present invention relates to a surface-modified elastic body, at least part of whose surface is modified by the above-described surface modification method.

The present invention relates to a medical device, at least part of whose surface is modified by the above-described surface modification method.

The present invention relates to a gasket for syringes, at least part of whose surface is modified by the above-described surface modification method.

The present invention relates to a catheter, at least part of whose surface is modified by the above-described surface modification method.

The present invention relates to a blood or body fluid analyzer, at least part of whose surface is modified by the above-described surface modification method.

### ADVANTAGEOUS EFFECTS OF INVENTION

The method for surface-modifying an object made of a rubber vulcanizate or a thermoplastic elastomer of the present invention includes: step 1 of forming polymerization initiation points on a surface of the object; step 2 of radically polymerizing a monomer starting from the polymerization initiation points to grow polymer chains on the surface of the

object; and step 3 of irradiating the grown polymer chains with an electron beam. Such a method makes it possible to impart excellent sliding properties, excellent durability after repeated sliding, and excellent properties to prevent adsorption or aggregation of proteins to the surface of the object, and further to improve and maintain the sealing properties for a long time. The resulting surface-modified elastic bodies have no PTFE polymer backbone, which permits sterilization by radiation such as $\gamma$ rays.

BRIEF DESCRIPTION OF DRAWINGS

Fig. 1 is an exemplary side view of an embodiment of a gasket for syringes.

DESCRIPTION OF EMBODIMENTS

The present invention relates to a method for surface-modifying an object made of a rubber vulcanizate or a thermoplastic elastomer, the method including: step 1 of forming polymerization initiation points on a surface of the object; step 2 of radically polymerizing a monomer starting from the polymerization initiation points to grow polymer chains on the surface of the object; and step 3 of irradiating the grown polymer chains with an electron beam.

Step 1 includes forming polymerization initiation points on a surface of a vulcanization-molded rubber or a molded thermoplastic elastomer (the object to be modified).

The rubber vulcanizate or thermoplastic elastomer may suitably contain a carbon atom adjacent to a double bond (i.e., allylic carbon atom).

Examples of rubber that can be used as the object to be modified include diene rubbers such as styrene-butadiene rubber, polybutadiene rubber, polyisoprene rubber, natural rubber, and deproteinized natural rubber; and butyl rubber and halogenated butyl rubber which have a degree of unsaturation of a few percent of isoprene units . The butyl rubber or halogenated butyl rubber, if used, is preferably cross-linked by triazine because the amount of matter extracted from the rubber vulcanizate is small. In this case, the rubber may contain an acid acceptor. Examples of suitable acid acceptors include hydrotalcite and magnesium carbonate.

If other rubbers are used, preferably sulfur vulcanization is performed. In this case, compounding ingredients commonly used for sulfur vulcanization may be added, such as vulcanization accelerators, zinc oxide, filler, and silane coupling agents. Suitable examples of the filler include carbon black, silica, clay, talc, and calcium carbonate.

The vulcanization conditions for the rubber may be appropriately chosen. The rubber is preferably vulcanized at 150°C or higher, more preferably 170°C or higher, still more preferably 175°C or higher.

Examples of the thermoplastic elastomer include polymer compounds that have rubber elasticity at room temperature owing to aggregates of plastic components (hard segments) serving as crosslinking points (e.g., thermoplastic elastomers (TPE) such as styrene-butadiene-styrene copolymer); and polymer compounds having rubber elasticity, obtained by mixing a thermoplastic component and a rubber component and dynamically crosslinking the mixture by a crosslinking agent (e.g., thermoplastic elastomers (TPV) such as polymer alloys containing a styrenic block copolymer or olefinic resin and a cross-linked rubber component).

Other examples of suitable thermoplastic elastomers include nylon, polyester, polyurethane, polypropylene, silicone, polyvinyl chloride, fluoroelastomers such as polytetrafluoroethylene (PTFE), and dynamically cross-linked thermoplastic elastomers thereof. Preferred among dynamically cross-linked thermoplastic elastomers are those obtained by dynamically crosslinking halogenated butyl rubber in a thermoplastic elastomer. This thermoplastic elastomer is preferably nylon, polyurethane, polypropylene, styrene-isobutylene-styrene block copolymer (SIBS), or the like.

The polymerization initiation points may be formed, for example, by adsorbing a polymerization initiator onto a surface of the object intended to be modified. Examples of the polymerization initiator include carbonyl compounds, organic sulfur compounds such as tetraethylthiuram disulfide, persulfides, redox compounds, azo compounds, diazo compounds, halogen compounds, and photoreductive pigments. Carbonyl compounds are preferred among these.

The carbonyl compound as the polymerization initiator is preferably benzophenone or its derivative, and may suitably be a benzophenone compound represented by the following formula:

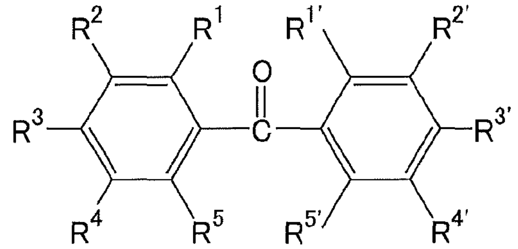

wherein $R^1$ to $R^5$ and $R^{1'}$ to $R^{5'}$ are the same as or different from one another and each represent a hydrogen atom, an alkyl group, a halogen (fluorine, chlorine, bromine, or iodine), a hydroxy group, a primary to tertiary amino group, a mercapto group, or a hydrocarbon group optionally containing an oxygen atom, a nitrogen atom, or a sulfur atom; and any two adjacent groups of $R^1$ to $R^5$ and $R^{1'}$ to $R^{5'}$ may be joined to each other to form a ring together with the carbon atoms to which they are attached.

Specific examples of the benzophenone compound include benzophenone, xanthone, 9-fluorenone, 2,4-dichlorobenzophenone, methyl o-benzoylbenzoate, 4,4'-bis(dimethylamino)benzophenone, and 4,4'-bis(diethylamino)benzophenone. Among these, benzophenone, xanthone, and 9-fluorenone are particularly preferred because then good polymer brushes can be formed. Other examples of suitable benzophenone compounds include fluorobenzophenone compounds, such as 2,3,4,5,6-pentafluorobenzophenone and decafluorobenzophenone.

Thioxanthone compounds can also be suitably used as the polymerization initiator because they provide high polymerization rate and also can easily be adsorbed on and/or reacted with rubber or the like. For example, compounds represented by the following formula can be suitably used.

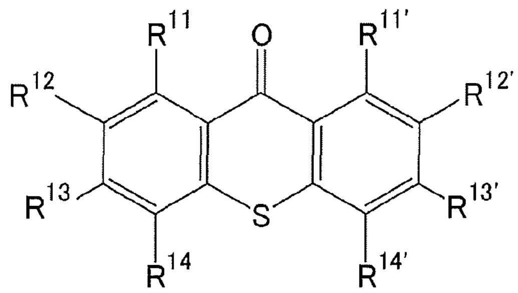

In the formula, $R^{11}$ to $R^{14}$ and $R^{11'}$ to $R^{14'}$ are the same as or different from one another and each represent a hydrogen atom, a halogen atom, an alkyl group, a cyclic alkyl group, an aryl group, an alkenyl group, an alkoxy group, or an aryloxy group.

Examples of thioxanthone compounds represented by the formula include thioxanthone, 2-isopropylthioxanthone, 4-isopropylthioxanthone, 2,3-dimethylthioxanthone, 2,4-dimethylthioxanthone, 2,3-diethylthioxanthone, 2,4-diethylthioxanthone, 2,4-dichlorothioxanthone, 2-methoxythioxanthone, 1-chloro-4-propoxythioxanthone, 2-cyclohexylthioxanthone, 4-cyclohexylthioxanthone, 2-vinylthioxanthone, 2,4-divinylthioxanthone, 2,4-diphenylthioxanthone, 2-butenyl-4-phenylthioxanthone, 2-methoxythioxanthone, and 2-p-octyloxyphenyl-4-ethylthioxanthone. Preferred among these are those which are substituted at one or two, particularly two, of $R^{11}$ to $R^{14}$ and $R^{11'}$ to $R^{14'}$ with alkyl groups. More preferred is 2,4-dimethylthioxanthone or 2,4-diethylthioxanthone.

The polymerization initiator such as a benzophenone compound or thioxanthone compound may be adsorbed onto the surface of the obj ect by known methods . When the polymerization initiator is a benzophenone compound or a thioxanthone compound, for example, the benzophenone or thioxanthone compound is dissolved in an organic solvent to prepare a solution; a surface portion of the object to be modified is treated with this solution so that the compound is adsorbed on the surface; and if necessary, the organic solvent is evaporated off by drying, whereby polymerization initiation points are formed. The surface may be treated by any method that allows the solution of the benzophenone or thioxanthone compound to be brought into contact with the surface of the object intended to be modified. Suitable methods may include applying or spraying the benzophenone or thioxanthone compound solution onto the surface; or, alternatively, immersing the surface into the solution. When only part of the surface needs to be modified, it is sufficient to adsorb the polymerization initiator only on the necessary part of the surface. In this case, for example, application or spraying of the solution is suitable. Examples of the solvent include methanol, ethanol, acetone, benzene, toluene, methyl ethyl ketone, ethyl acetate, and THF. Acetone is preferred because it does not swell the object intended to be modified and it can be rapidly dried and evaporated off.

Moreover, after the target portion to be modified is surface-treated with the benzophenone or thioxanthone compound solution so that the polymerization initiator is adsorbed, the polymerization initiator is preferably further chemically bonded to the surface of the object by irradiation with light. For example, the benzophenone or thioxanthone compound solution can be fixed to the surface by irradiation with ultraviolet light having a wavelength of 330 to 400 nm, preferably 350 to 390 nm. During step 1 and the fixing, hydrogen is abstracted from the rubber surface and the abstracted hydrogen is bonded to the oxygen atom in C=O to form C-O-H, as shown in the formula below. Moreover, since the hydrogen abstraction reaction selectively occurs on allylic hydrogen atoms in the object to be modified, the rubber preferably contains a butadiene or isoprene unit that contains an allylic hydrogen atom.

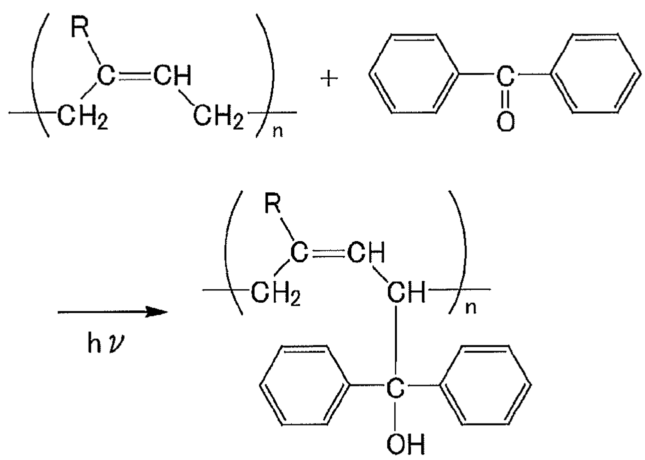

R: hydrogen or C1-C4 alkyl group

In particular, the polymerization initiator is preferably formed by treating the surface of the object with the polymerization initiator so that the polymerization initiator is adsorbed on the surface, and then irradiating the treated surface with LED light having a wavelength of 330 to 400 nm. Particularly preferably, after the surface of the object is treated with the benzophenone or thioxanthone compound solution so that the polymerization initiator is adsorbed, the adsorbed polymerization initiator is further chemically bonded to the surface by irradiating the treated surface with LED light having a wavelength of 330 to 400 nm. The LED light suitably has a wavelength of 350 to 390 nm.

Step 2 includes radically polymerizing a monomer starting from the polymerization initiation points formed in step 1 to grow polymer chains on the surface of the object intended to be modified. Thus, polymer chains radically polymerized from the monomer are formed on the surface of the object.

The monomer may be any conventionally known monomer. Suitable are, for example, those which allow the polymer chains grown in step 2 to form a crosslinked structure upon irradiation with an electron beam in step 3 which will be described later.

Specific examples of the monomer include (meth)acrylic acid, (meth)acrylic acid esters (e.g. methoxyethyl (meth)acrylate, hydroxyethyl (meth)acrylate), alkali metal salts of (meth) acrylic acid, amine salts of (meth) acrylic acid, and (meth) acrylonitrile. Monomers containing a C-N bond in the molecule may also be mentioned. Examples of monomers containing a C-N bond in the molecule include (meth) acrylamide; N-alkyl-substituted (meth)acrylamide derivatives such as N-ethyl(meth)acrylamide, N-n-propyl(meth)acrylamide, N-isopropyl(meth)acrylamide, N-cyclopropyl(meth)acrylamide, N-methoxymethyl(meth)acrylamide, N-methoxyethyl(meth)acrylamide, and N-ethoxyethyl(meth)acrylamide; N,N-dialkyl-substituted (meth)acrylamide derivatives such as N,N-dimethyl(meth)acrylamide, N,N-ethylmethyl(meth)acrylamide, and N,N-diethyl(meth)acrylamide; hydroxy(meth)acrylamide; hydroxy(meth)acrylamide derivatives such as N-hydroxyethyl(meth)acrylamide; and cyclic group-containing (meth) acrylamide derivatives such as (meth) acryloylmorpholine. Preferred among these are (meth)acrylic acid, (meth)acrylic acid esters, alkali metal salts of (meth)acrylic acid, amine salts of (meth)acrylic acid, acrylonitrile, (meth)acrylamide, dimethyl(meth)acrylamide, diethyl(meth)acrylamide, isopropyl(meth)acrylamide, hydroxyethyl(meth)acrylamide, and (meth)acryloylmorpholine. More preferred is acrylamide or acrylic acid.

In step 2, the monomer may be radically polymerized as follows. The (liquid) monomer or a solution thereof is applied (sprayed) to the surface of the object to which a benzophenone compound, a thioxanthone compound or the like is adsorbed or covalently bonded. Alternatively, the object is immersed in the (liquid) monomer or a solution thereof. Then, the object is irradiated with light, such as ultraviolet light having a wavelength of 330 to 400 nm, to allow the radical polymerization (photoradical polymerization) to proceed, whereby polymer chains are grown on the surface of the object intended to be modified. In another method, after the application, the surface may be covered with a transparent cover of glass, PET, polycarbonate, or the like, followed by irradiating the covered surface with light, such as ultraviolet light, to allow the radical polymerization (photoradical polymerization) to proceed, whereby polymer chains are grown on the surface of the object intended to be modified.

The solvent for application (spraying), the method for application (spraying), the method for immersion, the conditions for irradiation, and the like may be conventionally known materials or methods. The solution of the monomer (radically polymerizable monomer) may be an aqueous solution or a solution in an organic solvent that does not dissolve the polymerization initiator to be used, e.g. a benzophenone compound. Moreover, the (liquid) monomer or a solution thereof may contain a known polymerization inhibitor such as 4-methylphenol.

In the present invention, the radical polymerization of the monomer is allowed to proceed by light irradiation after the application of the (liquid) monomer or a solution thereof or after the immersion in the monomer or a solution

thereof. This may suitably be carried out by, for example, irradiation with a UV light source having an emission wavelength mainly in the ultraviolet region, such as a high pressure mercury lamp, metal halide lamp, or LED lamp, while blocking light of wavelengths equal to or shorter than 330 nm by a filter. The light dose may be appropriately chosen in view of polymerization time and uniformity of the reaction progress. Moreover, in order to prevent inhibition of polymerization due to active gas such as oxygen in the reaction vessel, oxygen is preferably removed from the reaction vessel and the reaction solution during or before the light irradiation. To this end, appropriate operations may be performed. For example, an inert gas, such as nitrogen gas or argon gas, is inserted into the reaction vessel and the reaction solution to discharge active gas such as oxygen from the reaction system and replace the atmosphere in the reaction system with the inert gas, or the reaction vessel is evacuated and degassed of oxygen. Furthermore, in order to prevent inhibition of the reaction due to oxygen or the like, appropriate adjustments may also be made, such as placing a UV light source so that an air layer (oxygen content: 15% or higher) does not exist between the reaction vessel made of glass, plastic or the like and the reaction solution or the object intended to be modified.

When irradiation with ultraviolet light is performed in step 2, the ultraviolet light preferably has a wavelength of 330 to 400 nm, more preferably 350 to 390 nm. Such ultraviolet light allows polymer chains to be formed well on the surface of the object intended to be modified. Irradiation with ultraviolet light having a wavelength of shorter than 330 nm can adversely cause the monomer to be polymerized independently of, not starting from, the surface to form free polymers. The light source may be a high-pressure mercury lamp, an LED with a center wavelength of 365 nm, an LED with a center wavelength of 375 nm, or the like. Irradiation with LED light having a wavelength of 330 to 400 nm, among others, is preferred; irradiation with LED light having a wavelength of 350 to 390 nm is more preferred. Particularly, LEDs and the like having a center wavelength of 365 nm, which is close to the excitation wavelength (366 nm) of benzophenone, are preferred in view of efficiency.

The polymer chains formed in step 2 provide excellent sliding properties, excellent durability, and excellent properties to prevent adsorption or aggregation of proteins, and further maintain good sealing properties. The polymerization degree of the formed polymer chains is preferably 20 to 200, 000, more preferably 350 to 50, 000. When the polymerization degree is less than 20, the polymer chains are so short that they may be concealed by the surface irregularities of the object, which tends to result in failure to provide sliding properties. When the polymerization degree is more than 200,000, the amount of monomer used increases, which tends to result in an economic disadvantage.

The polymer chains formed in step 2 preferably each have a length of 10 to 50,000 nm, more preferably 100 to 50,000 nm. When the length is shorter than 10 nm, good sliding properties tend not to be achieved. When the length is longer than 50,000 nm, a further improvement in sliding properties tends not to be expected while the cost of raw materials increases because the monomer used is expensive. In addition, in this case, surface patterns generated by the surface treatment tend to be visible to the naked eye and thereby spoil the appearance or reduce sealing properties.

In step 2, two or more types of monomers may simultaneously be radically polymerized starting from the polymerization initiation points. Moreover, multiple types of polymer chains may be grown on the surface of the obj ect intended to be modified.

Step 3 includes irradiating the polymer chains grown on the surface of the object in step 2 with an electron beam. This causes crosslinking between the polymer chains to give a surface-modified elastic body that can maintain good sealing properties for a long time as well as better sliding properties, durability, and properties to prevent adsorption or aggregation of proteins than those before the crosslinking, while at the same time, this step sterilizes the surface-modified elastic body. The details of the crosslinked structure formed in step 3 are not clear. Presumably, the electron beam irradiation produces radicals in the polymer chains, which cause crosslinks between the chains.

The acceleration voltage of the electron beam irradiated in step 3 is preferably 10 to 1,000 kV, more preferably 50 to 500 kV, still more preferably 100 to 200 kV.

The dose of the electron beam irradiated in step 3 is preferably 1 kGy or higher, while it is preferably 500 kGy or lower, more preferably 250 kGy or lower, still more preferably 100 kGy or lower, particularly preferably 30 kGy or lower. The electron beam at a dose of higher than 500 kGy may cause adverse effects such as molecular scission in the polymer chains or the object intended to be modified.

The source of the electron beam may be a known electron beam irradiator.

The polymer chains may be cross-linked to one another by ionic crosslinking, or crosslinking by a hydrophilic group containing an oxygen atom. Moreover, a small amount of a compound having at least two vinyl groups per molecule may be added and polymerized to introduce crosslinks between the polymer chains during the polymerization. The compound having at least two vinyl groups per molecule is preferably N,N'-methylenebisacrylamide or the like.

The surface modification method can be applied to rubber vulcanizates or thermoplastic elastomers to produce surface-modified elastic bodies. For example, surface-modified elastic bodies that are excellent in sliding properties in the presence of water or in a dry state can be obtained. These surface-modified elastic bodies are also excellent in that they have low friction and low water resistance or drag. Moreover, the method may be applied to at least part of a three-dimensional solid (e.g. elastic body) to obtain a surface-modified elastic body with modified properties . Furthermore, preferred examples of such surface-modified elastic bodies include polymer brushes. The polymer brush as used herein

means an assembly of graft polymer molecules obtained in the "grafting from" approach by surface-initiated living radical polymerization. Moreover, the graft chains are preferably oriented in a direction substantially vertical to the surface of the object intended to be modified because, in such a case, the entropy is reduced and thus the molecular mobility of the graft chains is reduced so that sliding properties are provided. Furthermore, semidilute or concentrated brushes having a brush density of 0.01 chains/$nm^2$ or higher are preferred.

The surface modification method can be applied to rubber vulcanizates or thermoplastic elastomers to prepare medical devices, e.g. a gasket for syringes, catheter, blood or body fluid analyzer (for example, a tube intended to extract blood or body fluid for diagnosis thereof), etc., at least part of whose surface is modified. Preferably, the surface of a medical device, such as a gasket for syringes, catheter, or blood or body fluid analyzer, may be modified at least at portions where sliding properties or lubricating properties are required or portions to come into contact with blood or body fluid. The entire surface may be modified.

Fig. 1 is an exemplary side view of an embodiment of a gasket for syringes. A gasket 1 shown in Fig. 1 has three circular protruding portions 11a, 11b and 11c which continuously protrude along the circumferential direction on the outer periphery that is to be in contact with the inner periphery of a syringe barrel. Examples of portions of the gasket 1 to which the surface modification is applied include: (1) the surfaces of protruding portions to be in contact with a syringe barrel, such as the circular protruding portions 11a, 11b and 11c; (2) the entire side surfaces including the circular protruding portions 11a, 11b and 11c; and (3) both a bottom surface 13 and the entire side surfaces.

EXAMPLES

The following will describe the present invention in more detail, referring to non-limiting examples.

(Example 1)

A chlorobutyl rubber containing isoprene units (degree of unsaturation: 1 to 2%) was cross-linked by triazine to prepare a vulcanized rubber gasket (vulcanized at 180°C for 10 minutes), which was then immersed in a 1 wt% solution of benzophenone in acetone so that benzophenone was adsorbed on the surface of the rubber vulcanizate. Thereafter, the rubber vulcanizate was taken out and dried.

The dried rubber vulcanizate was immersed in an aqueous acrylamide solution (1.25 M) in a glass reaction vessel, and irradiated with LED-UV light having a wavelength of 365 nm (2 mW/$cm^2$) for 150 minutes to cause radical polymerization and grow polymer chains on the surface of the rubber. Then, the surface was washed with water and dried.

The dried rubber vulcanizate was irradiated with an electron beam at an acceleration voltage of 150 kV and a dose of 20 kGy using an electron beam irradiator. In this manner, a surface-modified elastic gasket (a polymer brush) was obtained.

(Example 2)

A surface-modified elastic gasket was prepared as in Example 1, except that the dose of the electron beam was changed to 2 kGy.

(Example 3)

A surface-modified elastic gasket was prepared as in Example 1, except that the dose of the electron beam was changed to 200 kGy.

(Example 4)

A surface-modified elastic gasket was prepared as in Example 1, except that the aqueous acrylamide solution (1.25 M) was changed to a mixed solution of acrylamide and acrylic acid (1.25M, acrylamide: acrylic acid = 10 : 90), and the duration of LED-UV lamp irradiation was changed to 80 minutes.

(Example 5)

A surface-modified elastic gasket was prepared as in Example 4, except that the dose of the electron beam was changed to 5 kGy.

(Example 6)

A surface-modified elastic gasket was prepared as in Example 4, except that the dose of the electron beam was changed to 10 kGy.

(Example 7)

A surface-modified elastic gasket was prepared as in Example 4, except that the dose of the electron beam was changed to 50 kGy.

(Comparative Example 1)

A vulcanized rubber gasket prepared by crosslinking chlorobutyl rubber by triazine (vulcanized at 180°C for 10 minutes) was used.

The surface-modified elastic gaskets prepared in the examples and comparative example were evaluated by the methods described below. Table 1 shows the results.

(Length of polymer chain)

To determine the length of the polymer chain formed on the surface of each of the rubber vulcanizates, a cross section of the modified rubber body having polymer chains formed thereon was measured with an SEM at an acceleration voltage of 15 kV and a magnification of 1000 times. The thickness of the polymer layer photographed was determined and taken as the length of the polymer chain.

(Friction resistance)

To determine the friction resistance of the surface of the surface-modified elastic gaskets, the vulcanized rubber gaskets prepared in the examples and comparative example were each inserted into a COP resin barrel of a syringe and then pushed using a tensile tester while friction resistance was measured (push rate: 100 mm/min). The friction resistances of the examples and comparative example were converted to friction resistance index values using the equation below, with Comparative Example 1 set equal to 100. A lower index indicates lower friction resistance.

$$(\text{Friction resistance index}) = (\text{Friction resistance of each example})/(\text{Friction resistance of Comparative Example 1}) \times 100$$

[Table 1]

| | Example | | | | | | | Comparative Example |
|---|---|---|---|---|---|---|---|---|
| | 1 | 2 | 3 | 4 | 5 | 6 | 7 | 1 |
| Length of polymer chain (nm) | 6000 | 6000 | 6000 | 7500 | 7500 | 7500 | 7500 | - |
| Friction resistance index | 2.55 | 2.51 | 2.73 | 2.81 | 2.78 | 2.75 | 2.83 | 100 |

The results in Table 1 demonstrate that the surface of the surface-modified elastic bodies of the examples exhibited greatly reduced friction resistance and good sliding properties. These surface-modified elastic bodies were also comparable to Comparative Example 1 in terms of sealing properties. Further, these surface-modified elastic bodies maintained the sealing properties even after a long-term storage (at 40°C for 6 months) and showed better results than Comparative Example 1.

Thus, when these surface-modified elastic bodies are used as gaskets for syringe plungers, they provide sufficient sealing properties while reducing the friction of the plunger with the syringe barrel, and therefore they enable easy and accurate treatment with syringes. Also, if such polymer chains are formed on the inner surface of syringe barrels made of thermoplastic elastomers, treatment with the syringes can be readily performed as described above.

Furthermore, the above-mentioned effects can also be expected when such polymer chains are formed on the surface of the grooves formed on the tread or of the sidewalls of tires for use on passenger cars and other vehicles, on the surface of diaphragms, on the sliding surface of skis or snowboards, or on the surface of swimsuits, road signs, sign

boards, or the like.

REFERENCE SIGNS LIST

1: Gasket
11a, 11b, 11c: Circular protruding portion
13: Bottom surface

## Claims

1. A method for surface-modifying an object made of a rubber vulcanizate or a thermoplastic elastomer, the method comprising:

    step 1 of forming polymerization initiation points on a surface of the object;
    step 2 of radically polymerizing a monomer starting from the polymerization initiation points to grow polymer chains on the surface of the object; and
    step 3 of irradiating the grown polymer chains with an electron beam.

2. The method according to claim 1,
wherein the rubber vulcanizate or thermoplastic elastomer contains an allylic carbon atom which is adjacent to a double bond.

3. The method according to claim 1 or 2,
wherein the polymerization initiation points are formed by adsorbing a polymerization initiator onto the surface of the object.

4. The method according to claim 3,
wherein the polymerization initiator is at least one of a benzophenone compound or a thioxanthone compound.

5. The method according to claim 3 or 4,
wherein the adsorbed polymerization initiator is further chemically bonded to the surface of the object by irradiation with light.

6. The method according to any one of claims 1 to 5,
wherein the radical polymerization is photoradical polymerization.

7. The method according to claim 6,
wherein the photoradical polymerization involves irradiation with light having a wavelength of 330 to 400 nm.

8. The method according to any one of claims 1 to 7,
wherein the radical polymerization comprises inserting an inert gas into a reaction vessel and a reaction solution to replace an atmosphere therein with the inert gas before or during the light irradiation, and then polymerizing the monomer.

9. The method according to any one of claims 1 to 7,
wherein the radical polymerization comprises evacuating a reaction vessel to remove oxygen before or during the light irradiation, and then polymerizing the monomer.

10. The method according to any one of claims 1 to 9,
wherein the electron beam is at a dose of 1 to 500 kGy.

11. The method according to any one of claims 1 to 10,
wherein the monomer is at least one selected from the group consisting of acrylic acid, acrylic acid esters, acrylamide, alkali metal salts of acrylic acid, amine salts of acrylic acid, methacrylic acid, methacrylic acid esters, methacrylamide, alkali metal salts of methacrylic acid, amine salts of methacrylic acid, dimethylacrylamide, diethylacrylamide, isopropylacrylamide, hydroxyethylacrylamide, acryloylmorpholine, dimethylmethacrylamide, diethylmethacrylamide, isopropylmethacrylamide, hydroxyethylmethacrylamide, methacryloylmorpholine, and acrylonitrile.

**12.** The method according to any one of claims 1 to 11,
wherein the monomer or a solution thereof contains a polymerization inhibitor, and is polymerized in the presence of the polymerization inhibitor.

**13.** The method according to claim 12,
wherein the polymerization inhibitor is 4-methylphenol.

**14.** The method according to any one of claims 1 to 13,
wherein the polymer chains each have a length of 10 to 50,000 nm.

**15.** A surface-modified elastic body, at least part of whose surface is modified by the method according to any one of claims 1 to 14.

**16.** A medical device, at least part of whose surface is modified by the method according to any one of claims 1 to 14.

**17.** A gasket for syringes, at least part of whose surface is modified by the method according to any one of claims 1 to 14.

**18.** A catheter, at least part of whose surface is modified by the method according to any one of claims 1 to 14.

**19.** A blood or body fluid analyzer, at least part of whose surface is modified by the method according to any one of claims 1 to 14.

## Patentansprüche

**1.** Verfahren zum Oberflächenmodifizieren eines Objekts, das aus einem Kautschukvulkanisat oder einem thermoplastischen Elastomer hergestellt ist, wobei das Verfahren umfasst:

Schritt 1 eines Bildens von Polymerisationsinitiierungspunkten auf einer Oberfläche des Objekts;
Schritt 2 eines radikalischen Polymerisierens eines Monomers ausgehend von den Polymerisationsinitiierungspunkten, um Polymerketten auf der Oberfläche des Objekts wachsen zu lassen; und
Schritt 3 eines Bestrahlens der gewachsenen Polymerketten mit einem Elektronenstrahl.

**2.** Verfahren nach Anspruch 1, wobei das Kautschukvulkanisat oder thermoplastische Elastomer ein allylisches Kohlenstoffatom enthält, das benachbart zu einer Doppelbindung liegt.

**3.** Verfahren nach Anspruch 1 oder 2, wobei die Polymerisationsinitiierungspunkte durch Adsorbieren eines Polymerisationsinitiators auf der Oberfläche des Objekts gebildet werden.

**4.** Verfahren nach Anspruch 3, wobei der Polymerisationsinitiator zumindest einer ist von einer Benzophenonverbindung oder einer Thioxanthonverbindung.

**5.** Verfahren nach Anspruch 3 oder 4, wobei der adsorbierte Polymerisationsinitiator ferner durch Bestrahlung mit Licht chemisch an die Oberfläche des Objekts gebunden wird.

**6.** Verfahren nach einem der Ansprüche 1 bis 5, wobei die radikalische Polymerisation eine fotoradikalische Polymerisation ist.

**7.** Verfahren nach Anspruch 6, wobei die fotoradikalische Polymerisation mit einer Bestrahlung mit Licht mit einer Wellenlänge von 330 bis 400 nm einhergeht.

**8.** Verfahren nach einem der Ansprüche 1 bis 7, wobei die radikalische Polymerisation ein Einbringen eines Inertgases in ein Reaktionsgefäß und einer Reaktionslösung zum Ersetzen einer Atmosphäre darin durch das Inertgas vor oder während der Lichtbestrahlung und anschließend ein Polymerisieren des Monomers umfasst.

**9.** Verfahren nach einem der Ansprüche 1 bis 7, wobei die radikalische Polymerisation ein Evakuieren eines Reaktionsgefäßes zur Entfernung von Sauerstoff vor oder während der Lichtbestrahlung und anschließend ein Polymerisieren des Monomers umfasst.

**10.** Verfahren nach einem der Ansprüche 1 bis 9, wobei der Elektronenstrahl mit einer Dosis von 1 bis 500 kGy vorliegt.

**11.** Verfahren nach einem der Ansprüche 1 bis 10, wobei das Monomer zumindest eines ist, ausgewählt aus der Gruppe, bestehend aus Acrylsäure, Acrylsäureestern, Acrylamid, Alkalimetallsalzen von Acrylsäure, Aminsalzen von Acrylsäure, Methacrylsäure, Methacrylsäureestern, Methacrylamid, Alkalimetallsalzen von Methacrylsäure, Aminsalzen von Methacrylsäure, Dimethylacrylamid, Diethylacrylamid, Isopropylacrylamid, Hydroxyethylacrylamid, Acryloylmorpholin, Dimethylmethacrylamid, Diethylmethacrylamid, Isopropylmethacrylamid, Hydroxyethylmethacrylamid, Methacryloylmorpholin und Acrylnitril.

**12.** Verfahren nach einem der Ansprüche 1 bis 11, wobei das Monomer oder eine Lösung davon einen Polymerisationsinhibitor enthält und in Gegenwart des Polymerisationsinhibitors polymerisiert wird.

**13.** Verfahren nach Anspruch 12, wobei der Polymerisationsinhibitor 4-Methylphenol ist.

**14.** Verfahren nach einem der Ansprüche 1 bis 13, wobei die Polymerketten jeweils eine Länge von 10 bis 50.000 nm aufweisen.

**15.** Oberflächenmodifizierter elastischer Körper, von dem zumindest ein Teil seiner Oberfläche durch das Verfahren nach einem der Ansprüche 1 bis 14 modifiziert worden ist.

**16.** Medizinisches Gerät, von dem zumindest ein Teil seiner Oberfläche durch das Verfahren nach einem der Ansprüche 1 bis 14 modifiziert worden ist.

**17.** Dichtung für Spritzen, von der zumindest ein Teil ihrer Oberfläche durch das Verfahren nach einem der Ansprüche 1 bis 14 modifiziert worden ist.

**18.** Katheter, von dem zumindest ein Teil seiner Oberfläche durch das Verfahren nach einem der Ansprüche 1 bis 14 modifiziert worden ist.

**19.** Blut- oder Körperflüssigkeitsanalysator, von dem zumindest ein Teil seiner Oberfläche durch das Verfahren nach einem der Ansprüche 1 bis 14 modifiziert worden ist.

## Revendications

**1.** Procédé pour modifier en surface un objet réalisé en vulcanisat de caoutchouc ou en élastomère thermoplastique, le procédé comprenant :

une étape 1 consistant à former des points d'initialisation de polymérisation sur une surface de l'objet ;
une étape 2 consistant à faire une polymérisation radicalaire d'un monomère en partant des points d'initialisation de polymérisation pour faire pousser des chaînes polymères sur la surface de l'objet ; et
une étape 3 consistant à irradier les chaînes polymères qui ont poussé avec un faisceau d'électrons.

**2.** Procédé selon la revendication 1,
dans lequel le vulcanisat de caoutchouc ou l'élastomère thermoplastique contient un atome de carbone allylique qui est adjacent à une double liaison.

**3.** Procédé selon la revendication 1 ou 2,
dans lequel les points d'initialisation de polymérisation sont formés en adsorbant un initiateur de polymérisation sur la surface de l'objet.

**4.** Procédé selon la revendication 3,
dans lequel l'initiateur de polymérisation est au moins un composé parmi un composé benzophénone et un composé thioxanthone.

5. Procédé selon la revendication 3 ou 4,
dans lequel l'initiateur de polymérisation adsorbé est en outre adhéré chimiquement à la surface de l'objet par irradiation avec de la lumière.

6. Procédé selon l'une quelconque des revendications 1 à 5,
dans lequel la polymérisation radicalaire est une polymérisation photo-radicalaire.

7. Procédé selon la revendication 6,
dans lequel la polymérisation photo-radicalaire implique une irradiation avec de la lumière ayant une longueur d'onde de 330 à 400 nm.

8. Procédé selon l'une quelconque des revendications 1 à 7,
dans lequel la polymérisation radicalaire comprend l'introduction d'un gaz inerte dans un récipient de réaction et d'une solution de réaction pour remplacer une atmosphère dans celui-ci avec le gaz inerte avant ou pendant l'irradiation avec la lumière, puis la polymérisation du monomère.

9. Procédé selon l'une quelconque des revendications 1 à 7,
dans lequel la polymérisation radicalaire comprend l'évacuation d'un récipient de réaction pour supprimer l'oxygène avant ou pendant irradiation avec la lumière, puis la polymérisation du monomère.

10. Procédé selon l'une quelconque des revendications 1 à 9,
dans lequel le faisceau d'électrons a lieu à une dose de 1 à 500 kGy.

11. Procédé selon l'une quelconque des revendications 1 à 10,
dans lequel le monomère et au moins un choisi parmi le groupe constitué de acide acrylique, esters d'acide acrylique, acrylamide, sels de métaux alcalins d'acide acrylique, sels aminés d'acide acrylique, acide méthacrylique, esters d'acide méthacrylique, méthacrylamide, sels de métaux alcalins d'acide méthacrylique, sels aminés d'acide méthacrylique, diméthyle acrylamide, diéthyle acrylamide, isopropyle acrylamide, hydroxyéthyle acrylamide, acryloyle morpholine, diméthyle méthacrylamide, diéthyle méthacrylamide, isopropyle méthacrylamide, hydroxyéthyle méthacrylamide, méthacryloyle morpholine, et acrylonitrile.

12. Procédé selon l'une quelconque des revendications 1 à 11,
dans lequel le monomère ou une solution de celui-ci contient un inhibiteur de polymérisation, et est polymérisé en présence de l'inhibiteur de polymérisation.

13. Procédé selon la revendication 12,
dans lequel l'inhibiteur de polymérisation est du 4-méthyle phénol.

14. Procédé selon l'une quelconque des revendications 1 à 13,
dans lequel les chaînes polymères ont chacune une longueur de 10 à 50 000 nm.

15. Corps élastique modifié en surface, dont au moins une partie de la surface est modifiée par le procédé selon l'une quelconque des revendications 1 à 14.

16. Dispositif médical, dont au moins une partie de la surface est modifiée par le procédé selon l'une quelconque des revendications 1 à 14.

17. Joint pour seringue, dont au moins une partie de la surface est modifiée par le procédé selon l'une quelconque des revendications 1 à 14.

18. Cathéter, dont au moins une partie de la surface est modifiée par le procédé selon l'une quelconque des revendications 1 à 14.

19. Appareil d'analyse pour fluide sanguin ou corporel, dont au moins une partie de la surface est modifiée par le procédé selon l'une quelconque des revendications 1 à 14.

FIG.1

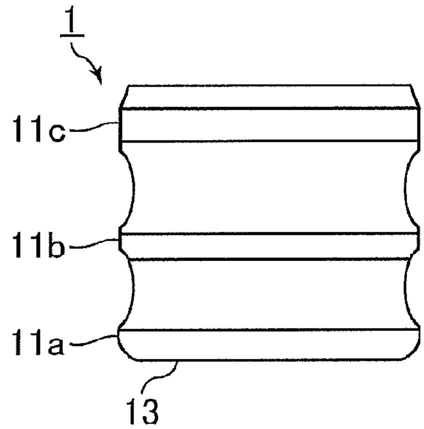

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012165525 A1
- CN 101565489 A
- JP 2001046956 A
- JP 2004298220 A
- JP 2010142573 A